# EUROPÄISCHE PATENTSCHRIFT

(11) **EP 1 006 106 B1**
(45) Veröffentlichungstag und Bekanntmachung des Hinweises auf die Patenterteilung: **09.02.2005**
(21) Anmeldenummer: 99123145.7
(22) Anmeldetag: 19.11.1999
(51) Int. Cl.: C07C 309/17, C11D 1/12, C08F 2/26

(54) **Benetzungsmittel, enthaltend sulfonierte Dicarbonsäureester**
Surfactants containing sulphonated dicarboxylic acid esters
Agents tensio-actifs contenant des esters dicarboxyliques sulfonés

(30) Priorität: 01.12.1998 DE 19855353
(43) Veröffentlichungstag der Anmeldung: 07.06.2000
(73) Patentinhaber: BASF AKTIENGESELLSCHAFT, 67056 Ludwigshafen (DE)
(72) Erfinder: Auchter, Gerhard, Dr., 67098 Bad Dürkheim (DE); Dragon, Andree, 67346 Speyer (DE); Aydin, Oral, Dr., 68165 Mannheim (DE); Wirsing, Friedrich, Dr., 67346 Speyer (DE)

(56) Entgegenhaltungen:
- DE-A- 4 432 363
- US-A- 2 121 616
- US-A- 2 121 617
- US-A- 6 110 381

## Beschreibung

Die Erfindung betrifft eine Lösung, enthaltend ein Salz eines Mono- oder Dialkylesters einer sulfonierten Dicarbonsäure a) und ein organisches Lösungsmittel b) mit einem Siedepunkt oberhalb 250°C bei 1 bar.

Weiterhin betrifft die Erfindung die Verwendung der Lösung als Benetzungsmittel insbesondere in wäßrigen Polymerdispersionen.

Wäßrige Polymerdispersionen werden z.B. als Lack oder als Klebstoff auf unterschiedlichste Substrate aufgetragen. Für eine gute Benetzung der Substrate und um eine möglichst glatte und störungsfreie Oberfläche zu erzielen enthalten die Polymerdispersionen im allgemeinen Benetzungsmittel (wetting agents).

Als Benetzungsmittel bekannt sind Dialkylester sulfonierter Bernsteinsäuren, z.B. aus D.J. Calvert et al., EUROPEAN ADHESIVES & SEALSANTS; Juni 1966, Seiten 2 bis 5.

Nachteilig bei der Verwendung von Benetzungsmitteln, insbesondere auch den Derivaten der sulfonierten Bernsteinsäure, ist eine starke Schaumbildung. Diese erreicht vor allem bei heutzutage üblichen, schnell laufenden Beschichtungsanlagen ein kritisches Ausmaß und verhindert die Ausbildung störungsfreier Oberflächen.

Aufgabe der vorliegenden Erfindung waren Benetzungsmittel, welche möglichst wenig zur Schaumbildung neigen.

Demgemäß wurde die oben definierte Lösung, ihre Verwendung als Benetzungsmittel sowie wäßrige Polymerdispersionen, welche die Benetzungsmittel enthalten, gefunden.

Die erfindungsgemäße Lösung enthält ein Salz eines Mono- oder Dialkylesters einer sulfonierten Dicarbonsäure, im nachfolgenden kurz mit a) bezeichnet.

Bei der Dicarbonsäure handelt es sich vorzugsweise um eine Dicarbonsäure mit 4 bis 8-C-Atomen, insbesondere handelt es sich um Bernsteinsäure (HOOC-CH₂-CH₂-COOH). Die Dicarbonsäure ist sulfoniert, d.h. durch mindestens eine, vorzugsweise eine Sulfonatgruppe substituiert.

Eine oder beide Carbonsäuregruppen sind mit Alkanolen verestert, so daß ein Mono- oder Dialkylester vorliegt. Die Alkylgruppen enthalten vorzugsweise 2 bis 20, besonders bevorzugt 4 bis 16, ganz besonders bevorzugt 4 bis 12 C-Atome.
Bevorzugt sind Dialkylester.
Besonders bevorzugt sind Di-C₄-C₁₂-alkylester, z.B. Di-octylester oder Di-2-ethylhexylester.

Bevorzugte Kationen des Salzes a) sind die Alkalikationen oder das Ammoniumkation. Insbesondere handelt sich bei a) daher um das Alkalisalz oder Ammoniumsalz eines Dialkylesters der sulfonierten Bernsteinsäure.

Als besonders bevorzugte Verbindung sei genannt:
Natrium-di-(2-ethylhexyl)-sulfosuccinat oder die entsprechende Kalium- oder Ammoniumverbindung.

Das organische Lösungsmittel b) ist bei 21°C und 1 bar flüssig und hat einen Siedepunkt oberhalb 250°C bei 1 bar. Das Lösungsmittel b) ist daher gemäß EU Direktive 96/13/EG (Amtsblatt der Europäischen Gemeinschaften Nr. L4 vom 06.01.1996, Seite 8 bis 13) eine nicht-flüchtige Verbindung.

Vorzugsweise ist das Lösungsmittel b) ein Tensid, d.h. eine Verbindung, welche die Oberflächenspannung von Wasser herabsetzt. Die Herabsetzung der Oberflächenspannung kann mit den bekannten Verfahren, z.B. durch Kontaktwinkelmessungen, bestimmt werden. Vorzugsweise ist b) aliphatisch.

Insbesondere handelt es sich bei b) um einen ein- oder mehrwertigen vorzugsweise einwertigen Alkohol, dessen Hydroxylgruppen alkoxyliert sind.

Bevorzugt sind Alkohole mit 10 - 20, insbesondere 12 bis 15 C-Atomen. Die Hydroxylgruppen sind im Mittel mit 3 bis 10 Mol Alkoxgruppen je mol Hydroxylgruppen alkoxyliert. In Betracht kommen insbesondere Propoxygruppen oder Ethoxygruppen. Besonders bevorzugt sind Ethoxygruppen.

Neben a) und b) kann die Lösung weitere Bestandteile haben. In Betracht kommt insbesondere Wasser als Verdünnungsmittel bzw. Hilfslösemittel.

Vorzugsweise ist a) bei Normalbedingungen (21°C, 1 bar) in b) löslich, wobei die Löslichkeit insbesondere mindestens 20 Gew.-Teile a) in 100 Gew.-Teilen b) beträgt.

Bevorzugte Lösungen enthalten 50 bis 500, besonders bevorzugt 100 bis 400 und ganz besonders bevorzugt 200 bis 350 Gew.-Teile a), bezogen auf 100 Gew.-Teile b).

Wasser wird, wenn überhaupt, vorzugsweise in Mengen von 1 bis 300, besonders bevorzugt von 100 bis 200 Gew.-Teilen, bezogen auf 100 Gew.-Teile b) mitverwendet.

Die Lösung kann in einfacher Weise durch Mischen der Bestandteile hergestellt werden. Vorzugsweise wird zunächst a) in b) gelöst und dann, falls gewünscht, Wasser zur Verdünnung zugesetzt.

Die Lösung eignet sich als Benetzungsmittel. Insbesondere eignet sich die Lösung als Benetzungsmittel für Polymere, vorzugsweise solche die durch radikalische Polymerisation erhältlich sind.

Die Polymere bestehen vorzugsweise zu mindestens 60 Gew.-% aus C₁-C₁₈-Alkylacrylaten oder Akylmethyacrylaten, Vinylaromaten mit bis zu 20 C-Atomen, Vinylestern von C₁-C₂₀-Carbonsäure, ethylenisch ungesättigten Nitrilen, Vinylhalogeniden, Vinylethern von 1 bis 10 c-Atome enthaltenden Alkoholen und aliphatischen Kohlenmonomerstoffen mit 2 bis 8-C-Atomen und 1 oder 2 Doppelbindungen oder Mischungen dieser Monomere.

Bevorzugt sind Polymere, welche zu mindestens 60 Gew.-% aus C₁-C₁₈-Alkylacrylaten oder Alkylmethacrylaten, aus Vinylaromaten mit bis zu 20 C-Atomen oder aliphatischen, konjugierten Dienen mit 4 bis 8 C-Atomen oder Mischungen dieser Monomeren bestehen.

Die Polymeren liegen vorzugsweise in Form einer wäßrigen Dispersion vor, wie sie z.B. durch Emulsionspolymerisation der Monomeren erhalten wird.

Die Lösung wird als Benetzungsmittel vorzugsweise in Mengen von 0,1 bis 10, besonders bevorzugt 0,2 bis 5, ganz besonders bevorzugt 0,2 bis 3 Gew.-Teilen Lösung je 100 Gew.-Teilen Polymer verwendet.

Im Fall der wäßrigen Polymerdispersionen wird die erfindungsgemäße Lösung einfach der Dispersion zugesetzt und verrührt.

Die erfindungsgemäße Lösung kann auch als Emulgator z.B. bei der Emulsionspolymerisation verwendet werden.

Dazu werden die Monomeren mit Hilfe der erfindungsgemäßen Lösung in Wasser dispergiert und anschließend die Emulsionspolymerisation in bekannter Weise durchgeführt. Die Gew.-Teile der erfindungsgemäßen Lösung im Fall der Verwendung als Emulgator entsprechen den oben angegebenen.

Bei der Auftragung der Polymeren bzw. der Polymerdispersionen auf unterschiedlichste Substrate, z.B. aus Kunststoff, Metall, Holz, Papier, wirkt die Lösung als Benetzungsmittel und führt zur Ausbildung einer glatten und störungsfreien Polymeroberfläche. Insbesondere auch bei flexiblen Substraten, wie Kunststofffolien oder silikonisiertem Papier können sehr gute Oberflächen erhalten werden.

Die Lösung enthält keine flüchtigen organischen Lösemittel mit einem Siedepunkt unter 250°C (1 bar).

Bisherige Benetzungsmittel verursachen bei ihrer Verwendung im wäßrigen Polymerdispersionen während des Auftragens auf Oberflächen oft eine Schaumbildung. Insbesondere bei der Verwendung von wäßrigen Polymerdispersionen als Klebstoff, z.B. bei der Beschichtung von Etiketten, konnten so keine gleichmäßigen Oberflächen erhalten werden.

Mit der erfindungsgemäßen Lösung ist eine Schaumbildung auch auf schnell laufenden Beschichtungsanlagen, (z.B. zur Beschichtung von Etiketten) nicht oder kaum zu beobachten.

### Beispiele

### I. Benetzungsmittel (B)

### B1 (erfindungsgemäß)

Lösung aus

| | |
|---|---|
| 71 Gew.-% | Natrium-di(2-ethylhexyl)sulfosuccinat (DOSS) |
| 29 Gew.-% | eines mit durchschnittlich 5 Mol Ethylenoxid (EO) ethoxylierten C₁₃-Alkohol (EO-Alkohol) |

(B1 wurde noch mit 45 Gew.-Teilen Wasser verdünnt, welche aber nicht zur Gewichtsmenge des Benetzungsmittels zugerechnet werden).

### B2 (zum Vergleich)

100 Gew.-% DOSS
(wurde in Wasser/Propylenglykolgemisch gelöst, welches aber nicht zur Gewichtsmenge des Benetzungsmittels zugerechnet wird).

### B3 (zum Vergleich)

100 Gew.-% EO-Alkohol

### II. Prüfmethoden und Ergebnisse

Die Benetzungsmittel wurden zu einer Haftklebstoffmischung aus 75 Teilen (bezogen auf Feststoff) einer handelsüblichen Acrylat-Haftklebstoffdispersion (Acronal® V210, Hersteller BASF AG, Ludwigshafen, Deutschland) und 25 Teilen (bezogen auf Feststoff) einer wässrigen Kolophoiumharzdispersion (Tacolyn (® 3179, Hersteller Hercules BV, Middelburg, Niederlande) gegeben. (Menge in Tabelle)

Danach wurden die Klebstoffe mit Wasser auf eine Auslaufzeit von 19 Sekunden, gemessen im DIN-4-Auslaufbecher nach DIN 53211, eingestellt.

### Prüfung des Benetzungsverhaltens

Die Klebstoffmischungen werden mit einer Kastenrakel mit 50 µm Spalthöhe auf silikonisiertes Papier aufgetragen. Nach einer Wartezeit von 10 s wird die benetzte Fläche in Prozent der ursprünglich mit Dispersion beschichteten Fläche bestimmt durch Abschätzung. Das Benetzungsverhalten ist umso besser, je größer die noch benetzte Fläche nach diesem Zeitraum ist. In der Tabelle angegeben ist die benetzte Fläche in % als Mittelwert einer 5maligen Bestimmung.

### Prüfung des Schaumverhaltens

- Prüfgerät:: Becherglas 1000 ml (Höhe 14,5 cm, Durchmesser 10,5 cm)
Korbrührer (Höhe 5,5 cm, Durchmesser 4,1 cm) mit 15 Reihen von je 20 versetzt angeordneten Löchern (Lochdurchmesser 3 mm, Lochabstand 3 mm) teflonbesetzter Metallschaber
- Ausgangsvolumen:: 500 ml

### Verfahrensweise:

In das Becherglas werden 500 ml Dispersion eingefüllt. Der Korbrührer wird so in die Dispersion eingetaucht, daß sich die oberen drei Lochreihen des Rührers außerhalb der Dispersion befinden. Im Winkel von etwa 45 Grad zum Rührer wird ein teflonbesetzter Metallschaber angesetzt und der Rührer mit 500 Umdrehungen pro Minute gedreht. Nach festgelegten Zeitabständen wird nun der Anstieg des Schaumvolumens im Becherglas gemessen. In der Tabelle angegeben ist die Schaumhöhe nach 120 Minuten.

**Tabelle**

| Versuchs Nr. | Benetzungsmittel | Menge* Gew.-% | Benetzte Fläche % | Schaumhöhe |
|---|---|---|---|---|
| 1 | B1 | 0,8 | 60 | 750 ml |
| 2 | B2 | 0,57** | 45 | 750 ml |
| 3 | B2 | 0,8 | 65 | > 1000 ml*** |
| 4 | B3 | 0,8 | 25 | -- |

| | | | | |
|---|---|---|---|---|
| * bezogen auf Feststoffmenge des Klebstoffs | | | | |
| ** entspricht gleicher DOSS-Menge wie in Versuch Nr. 1 | | | | |
| *** bereits nach 82 Minuten | | | | |

Insbesondere ein Vergleich von Versuch 1 mit 3 macht deutlich, daß erfindungsgemäß die Schaumbildung reduziert werden kann und bei gleicher Menge des Benetzungsmittels (trotz deutlich geringerer DOSS Menge) die Benetzungswirkung gleich bleibt.

## Patentansprüche

1. Lösung, enthaltend ein Salz eines Mono- oder Dialkylesters einer sulfonierten Dicarbonsäure a) und ein organisches Lösungsmittel b) mit einem Siedepunkt oberhalb 250°C bei 1 bar.

2. Lösung gemäß Anspruch 1, wobei es sich bei a) um einen Dialkylester handelt.

3. Lösung gemäß Anspruch 1 oder 2, wobei es sich bei a) um einen Dialkylester der sulfonierten Bernsteinsäure handelt.

4. Lösung gemäß einem der Ansprüche 1 bis 3, wobei es sich bei a) um ein Alkalisalz oder ein Ammoniumsalz des Dialkylesters der sulfonierten Bernsteinsäure handelt.

5. Lösung gemäß einem der Ansprüche 1 bis 4, wobei es sich bei dem Lösungsmittel b) um ein Tensid handelt.

6. Lösung gemäß einem der Ansprüche 1 bis 5, wobei es sich bei dem Lösungsmittel b) um alkoxylierte Alkohole handelt.

7. Lösung gemäß einem der Ansprüche 1 bis 6, enthaltend 50 bis 500 Gew.-teile der Verbindung a), bezogen auf 100 Gew.-teile des Lösungsmittels b).

8. Verwendung der Lösung gemäß einem der Ansprüche 1 bis 7 als Benetzungsmittel in wäßrigen Polymerdispersionen.

9. Verwendung der Lösung gemäß einem der Ansprüche 1 bis 7 als Emulgator bei der Emulsionspolymerisation.

10. Wäßrige Polymerdispersionen, enthaltend eine Lösung gemäß einem der Ansprüche 1 bis 7.

11. Verwendung der wäßrigen Polymerdispersion gemäß Anspruch 9 zur Beschichtung von Kunststofffolien oder silikonisiertem Papier.

## Claims

1. A solution comprising a salt of a monoalkyl or dialkyl ester of a sulfonated dicarboxylic acid a) and an organic solvent b) having a boiling point of more than 250°C at 1 bar.

2. A solution as claimed in claim 1, wherein a) is a dialkyl ester.

3. A solution as claimed in claim 1 or 2, wherein a) is a dialkyl ester of sulfonated succinic acid.

4. A solution as claimed in any of claims 1 to 3, wherein a) is an alkali metal salt or an ammonium salt of the dialkyl ester of sulfonated succinic acid.

5. A solution as claimed in any of claims 1 to 4, wherein the solvent b) is a surfactant.

6. A solution as claimed in any of claims 1 to 5, wherein the solvent b) is an alkoxylated alcohol.

7. A solution as claimed in any of claims 1 to 6, containing from 50 to 500 parts by weight of compound a) per 100 parts by weight of solvent b).

8. The use of a solution as claimed in any of claims 1 to 7 as a wetting agent in an aqueous polymer dispersion.

9. The use of a solution as claimed in any of claims 1 to 7 as an emulsifier in emulsion polymerization.

10. An aqueous polymer dispersion comprising a solution as claimed in any of claims 1 to 7.

11. The use of an aqueous polymer dispersion as claimed in claim 9 to coat polymer films or siliconized paper.

## Revendications

1. Solution contenant un sel d'un ester mono- ou dialkylique d'un acide dicarboxylique sulfoné a) et un solvant organique b) d'un point d'ébullition supérieur à 250°C sous 1 bar.

2. Solution selon la revendication 1, dans laquelle a) est un ester dialkylique.

3. Solution selon la revendication 1 ou 2, dans laquelle a) est un ester dialkylique d'acide succinique sulfoné.

4. Solution selon l'une quelconque des revendications 1 à 3, dans laquelle a) est un sel de métal alcalin ou un sel d'ammonium d'ester dialkylique d'acide succinique sulfoné.

5. Solution selon l'une quelconque des revendications 1 à 4, dans laquelle le solvant b) est un tensioactif.

6. Solution selon l'une quelconque des revendications 1 à 6, dans laquelle le solvant b) est un alcool alcoxylé.

7. Solution selon l'une quelconque des revendications 1 à 6, contenant de 50 à 500 parties en poids du composé a), par rapport à 100 parties en poids du solvant b).

8. Utilisation de la solution selon l'une quelconque des revendications 1 à 7 comme agent mouillant dans des dispersions aqueuses de polymères.

9. Utilisation de la solution selon l'une quelconque des revendications 1 à 7 comme émulsifiant lors de la polymérisation en émulsion.

10. Dispersions aqueuses de polymères contenant une solution selon l'une quelconque des revendications 1 à 7.

11. Utilisation de la dispersion aqueuse de polymère selon la revendication 9 pour le revêtement de feuilles de plastiques ou de papier siliconé.
